# EUROPEAN PATENT APPLICATION

(11) **EP 4 647 009 A1**
(43) Date of publication of application: **12.11.2025**
(21) Application number: 25175319.0
(22) Date of filing: 09.05.2025
(51) Int. Cl.: A61B 5/339, A61B 5/367, A61B 5/00

(54) **CARDIAC MAP DIMMING WITH GUI**

(30) Priority: 10.05.2024 US 202463645326 P; 27.03.2025 US 202519091931
(71) Applicant: Biosense Webster (Israel) Ltd., 2066717 Yokneam (IL)
(72) Inventor: SEGEV, Meytal, 2066717 Yokneam (IL); YANOVICH, Nir, 2066717 Yokneam (IL); MASSARWA, Fady, 2066717 Yokneam (IL)
(74) Representative: Carpmaels & Ransford LLP

(57) **Abstract**

A system included an input device and a processor. The processor is configured to (i) receive or generate an electrophysiological (EP) map comprising one or more regions of interest (ROIs), (ii) receive from a user, via the input device, a level of deemphasis the EP map required outside the ROIs, (iii) deemphasize the EP map outside the ROIs responsively to the required level, and (iv) display the deemphasized EP map to the user.

## Description

### CROSS-REFERENCE TO RELATED APPLICATION

This application claims the benefit of U.S. Provisional Patent Application 63/645,326, filed May 10, 2024, which is incorporated herein by reference.

### FIELD OF THE DISCLOSURE

The present invention relates generally to electrophysiological mapping, and particularly to visualization of cardiac electrophysiological data points and maps.

### BACKGROUND OF THE DISCLOSURE

An electrophysiological (EP) map of a cardiac chamber of a patient is generated by positioning electrodes on a region of the chamber's tissue, acquiring an EP signal of the region, and then repeating the process for a different region. EP parameters are extracted from the EP signals in each region of measurement and then displayed over a graphical representation of the tissue, such as a three-dimensional (3D) rendering of the cardiac chamber.

EP mapping visualization methods previously proposed in the patent literature to ease an interpretation of an EP map. For example, U.S. Patent 11,304,645 describes methods, apparatus, and systems for medical procedures that include receiving a first set of biometric data for a first portion of a body part and determining a first range of values in the first set of biometric data, determining first visual characteristics based on the first range of values and rendering a global view of the first portion of the body part rendered with the first visual characteristics. A second range of values in a second set of biometric data for a second portion of the body part may be determined and the second portion of the body part may be a subset of the first portion of the body part. Second visual characteristics may be determined based on the second range of values and a local view including the second portion of the body part rendered with the second visual characteristics may be rendered.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electrophysiological (EP) mapping and ablation system, in accordance with an example of the present disclosure;
Fig. 2 is a schematic illustration of a graphical user interface (GUI) that lets a user adjust a level of dimming of an EP map outside ROIs on an EP map, in accordance with an example of the present disclosure;
Figs. 3A-3C are schematic, pictorial volume renderings of an LAT map of a cardiac chamber with an increased level of dimming of an EP map outside of ROIs, in accordance with an example of the present disclosure; and
Fig. 4 is a flow chart that schematically illustrates a method for using a GUI and an input device to deemphasize an EP map outside ROIs, in accordance with an example of the present disclosure.

### DETAILED DESCRIPTION OF EXAMPLES

### OVERVIEW

In a typical electrophysiological (EP) mapping procedure, a mapping system generates an EP map of a cardiac chamber based on EP data captured at a plurality of locations inside a cardiac chamber. In the procedure, a mapping system applies catheters to sense the activation wave (reference signal) and the resulting activations (substrate signal). A processor annotates the reference signal and the respective substrate activation. Typically, hundreds of waveforms are acquired and annotated during a mapping procedure. Using the annotations, the processor calculates the respective EP value, for example, a local activation time (LAT) value between the reference signal and the respective substrate signal.

Using the calculated EP values (e.g., LAT values, bipolar amplitude, etc.), the processor can generate a respective EP map, such as an LAT map or a bipolar voltage map. The EP map is a useful tool in the diagnosis of an arrhythmia (e.g., atrial fibrillation) to guide a physician to eliminate it, e.g., using ablation.

In some cases, however, the EP map may be hard to interpret due to its visual load, especially outside a region of interest (ROI) which shows an area suspected as arrhythmogenic. This visual load makes it difficult for the physician to focus on the ROIs and make a clinical decision (e.g., decide where to ablate).

In some cases, the processor of the EP mapping system also generates automatic tags of other EP parameters (e.g., signal fractionation count) over the EP map, which increases the difficulty to interpret the map due to (i) a large number of tags, or tags with different colors, presented simultaneously, (ii) tag colors possibly merging with the coloring of a substrate map (e.g., LAT map, bipolar potential map), and (iii) the aforementioned visual load of information outside ROIs.

The ROIs can represent another designated signal with clinical significance, such as fractionated signals or late potentials.

Examples of the present invention that are described hereinafter provide a technique to reduce visual overload so a physician can make better use of the EP map during a clinical procedure.

The disclosed technique provides new visualization modes by (i) allowing the user to select and adjust the level of darkening (dimming effect) of an EP map outside ROIs, and (ii) highlighting ROIs (with or without tags being presented therein). Further, the technique lets an activation wave propagation mode of the display show the activation wave in highlighted areas of an interest mode.

The disclosed technique allows the user to concentrate fully on ROIs without visual distractions that might be caused by overwhelming coloring and/or number of tags. In particular, the option to control the visibility level of non-highlighted (e.g., dimmed) EP map areas enable the user to focus on ROIs and, at the same time, adjust the coloring and propagation of the area outside the ROIs according to user needs.

In an example, a system is provided that includes a display device, an input device (e.g., a touch screen or mouse), and a processor. The processor is configured to receive or generate an EP map comprising one or more ROIs. The processor is further configured to receive from the user, via the input device, a level of deemphasis the EP map required outside the ROIs. Responsively to the requirement, the processor deemphasizes the EP map outside the ROIs and displays the visually modified map to the user. For example, the disclosed technique assists the physician to accurately locate a catheter, within an ROI of a challenging 3D visualization, in order to perform an ablation.

The processor may deemphasize the EP map outside the ROIs by, for example, dimming the map outside the ROIs. The user might be able to apply the highlighted zones using design lines that he manually applies.

In another example, using the display device and the input device, the processor is configured to provide a graphical user interface (GUI) feature that lets a user deemphasize the EP map outside the ROIs by, for example, adjusting a required dimming level using a sliding ruler scale of the GUI.

Concerning the ROIs, in some examples, further visualization is calculated and presented around the automated detected tags to enable, for example, a unique highlight (e.g., using contouring for emphasis) of the ROIs. The ROI visualization assists the physician to distinguish specific ROIs (e.g., with fractionated signals or late signals) to build an ablation strategy.

### SYSTEM DESCRIPTION

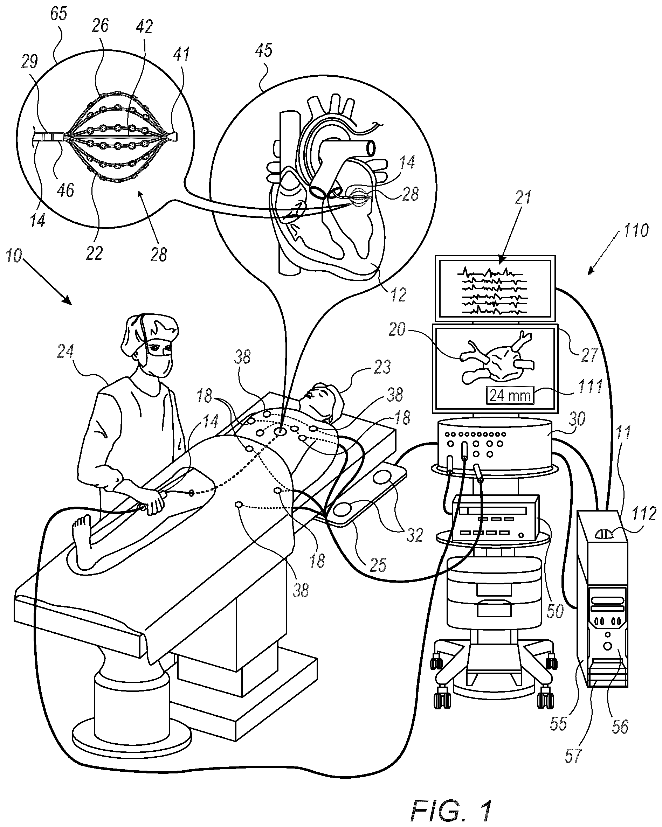

Fig. 1 is a schematic, pictorial illustration of a catheter-based electrophysiological (EP) mapping and ablation system 10, in accordance with an example of the present disclosure.

System 10 includes multiple catheters which are percutaneously inserted by physician 24 through the patient's vascular system into a chamber or vascular structure of a heart 12 (seen in inset 45). Typically, a delivery sheath catheter is inserted into a cardiac chamber, such as a ventricle or an atrium, near a desired location in heart 12. Thereafter, a plurality of catheters is inserted into the delivery sheath catheter to arrive at the desired location. The plurality of catheters may include a catheter dedicated for pacing, a catheter for sensing intracardiac electrogram signals, a catheter dedicated for ablating and/or a catheter dedicated for both EP mapping and ablating. An example catheter 14, illustrated herein, is configured for sensing bipolar electrograms. Physician 24 brings a distal tip 28 (also called hereinafter distal end assembly 28) of catheter 14 into contact with the heart wall for sensing a target site in heart 12. For ablation, physician 24 similarly brings a distal end of an ablation catheter to a target site.

As seen in inset 65, catheter 14 is an exemplary catheter that includes a basket distal end 28, including one, and preferably multiple, electrodes 26 optionally distributed over a plurality of splines 22 at distal tip 28 and configured to sense IEGM signals. Catheter 14 may additionally include a position sensor 29 embedded in or near distal tip 28 on a shaft 46 of catheter 14, used to track the position and orientation of distal tip 28. Optionally, and preferably, position sensor 29 is a magnetic-based position sensor including three magnetic coils for sensing three-dimensional (3D) position and orientation. As seen, distal tip 28 further includes an expansion/collapse rod 42 of expandable assembly 28 that is mechanically connected to basket assembly 28 at a distal edge 41 of assembly 28.

Magnetic based position sensor 29 may be operated together with a location pad 25 that includes a plurality of magnetic coils 32 configured to generate magnetic fields in a predefined working volume. Real-time position of distal tip 28 of catheter 14 may be tracked based on magnetic fields generated with location pad 25 and sensed by magnetic based position sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays, on display device 27, cardiac signals 21 (e.g., electrograms acquired at respectively tracked cardiac tissue positions) acquired with body surface ECG electrodes 18 and intracardiac electrograms acquired with electrodes 26 of catheter 14. Recorder 11 may include pacing capability to pace the heart rhythm, and/or may be electrically connected to a standalone pacer.

Workstation 55 includes memory 57, a processor 56 unit with memory or storage with appropriate operating software loaded therein, and user interface capability. Workstation 55 may provide multiple functions, optionally including (i) modeling endocardial anatomy in three-dimensions (3D) and rendering the model or EP map 20 for display on display device 27, (ii) displaying on display device 27 activation sequences (or other data) compiled from recorded cardiac signals 21 in representative visual indicia or imagery superimposed on the rendered EP map 20, (iii) displaying real-time location and orientation of multiple catheters within the heart chamber, and (iv) displaying sites of interest on display device 27, such as places where ablation energy has been applied. One commercial product embodying elements of system 10 is available as the CARTO^{™} 3 System, available from Biosense Webster, Inc., 31A Technology Drive, Irvine, CA 92618.

In a disclosed example, physician 24 uses a GUI 111 to deemphasize (e.g., dim using an input device such as mouse 112) EP map 20 outside ROIs predetermined on the EP map.

System 10 may include an ablation energy generator 50 that is adapted to conduct ablative energy to one or more electrodes at a distal tip of a catheter configured for ablation. Energy produced by ablation energy generator 50 may include, but is not limited to, radiofrequency (RF) energy or pulsed-field ablation (PFA) energy, including monopolar or bipolar high-voltage DC pulses, to be used to effect irreversible electroporation (IRE), or combinations thereof.

Patient interface unit (PIU) 30 is an interface configured to establish electrical communication between catheters, electrophysiological equipment, power supply and a workstation 55 to control system 10 operation and to receive EA signals from the catheter. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

In some examples, processor 56 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

This configuration of system 10 is shown by way of example, to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other types of medical systems. For example, other multi-electrode catheter types may be used, such as the multi-arm OCTARAY^{™} catheter or a flat catheter.

### GUI FOR EP MAP WITH DIMMED AREA OUTSIDE ROIS

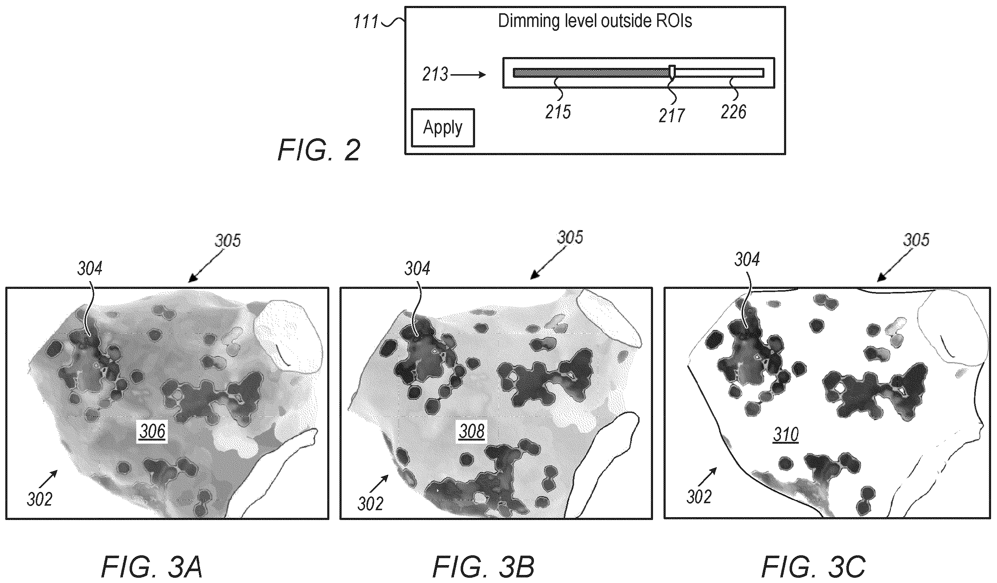

Fig. 2 is a schematic illustration of GUI 111 that lets a user adjust a level 215 of dimming of EP map 302 outside ROIs on the EP map, in accordance with an example of the present disclosure.

In the shown example, GUI 111 comprises a sliding ruler 213 having a full range 226 from entirely darkening to fully brightening the EP map outside ROIs. The user can adjust dimming level 215 within full range 226 by sliding a separator 217.

### EP MAP DISPLAYING DIMMED AREA OUTSIDE ROIS

Figs. 3A-3C are schematic, pictorial volume renderings of an LAT map 302 of a cardiac chamber with an increased level of dimming of the EP map outside (305) ROIs 304, in accordance with an example of the present disclosure.

In Fig. 3A the user selects a low dimming level 306, such that the LAT map area 305 outside the ROIs 304 is relatively visible. At the same time, ROIs 304 are sufficiently distinct.

In Fig. 3B the user selects a mid-dimming level 308, such that the LAT map area 305 outside the ROIs 304 is still visible, though ROIs 304 are dominantly distinct.

In Fig. 3C the user selects a max dimming level 310, such that the LAT map area 305 outside the ROIs 304 is invisible, and the user sees only ROIs 304.

While Fig. 3 describes a LAT map, the description is applicable to other EP maps, such as to bipolar potential map.

### METHOD TO DEEMPHASIZE EP MAP OUTSIDE ROIS

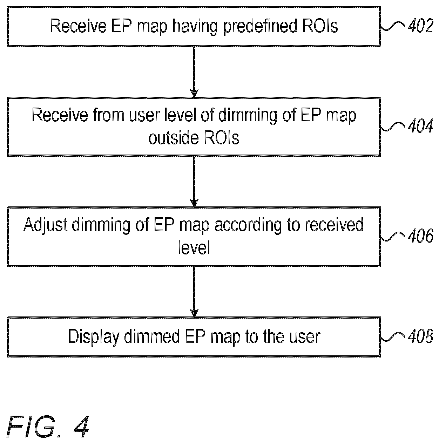

Fig. 4 is a flow chart that schematically illustrates a method for using GUI 111 and input device 112 to deemphasize EP map 302 outside (305) ROIs 304, in accordance with an example of the present disclosure.

The process carries out an algorithm that begins with processor 56 receiving EP map 302 having predefined ROIs 304, at EP map receiving step 402.

At a dimming level receiving step 404, the processor receives from the user, via input device 112 (e.g., by sliding separator 217), a required dimming level (e.g., darkening) on GUI 111 of Fig. 2 of EP map 302 outside regions 304.

The processor adjusts the level of dimming according to the required dimming level received (e.g., one of dimming levels 306, 308, and 310), at setting EP map dimming step 406.

At EP map displaying step 408, the processor displays the dimmed EP map 302 to the user.

### EXAMPLES

### Example 1

A system (10) included an input device (110) and a processor (56). The processor (56) is configured to (i) receive or generate an electrophysiological (EP) map (302) comprising one or more regions of interest (ROIs) (304), (ii) receive from a user, via the input device (112), a level of deemphasis (306, 308, 310) the EP map (302) required outside (305) the ROIs (304), (iii) deemphasize the EP map outside the ROIs responsively to the required level (215, 306, 308, 310), and (iv) display the deemphasized EP map to the user.

### Example 2

The system (10) according to example 1, wherein the processor (56) is configured to deemphasize the EP map (302) outside (305) the ROIs by darkening the EP map outside the ROIs.

### Example 3

The system (10) according to any of examples 1 and 2, wherein the processor (56) is further configured to provide, using a display device (27) and the input device (110), a graphical user interface (GUI) (111) feature that lets the user adjust the level of deemphasis.

### Example 4

The system (10) according to any of examples 1 through 3, wherein the GUI (111) feature comprises a sliding ruler (213) that lets the user adjust the level of the deemphasis.

### Example 5

The system (10) according to any of examples 1 through 4, wherein the EP map (302) is a local activation time (LAT) map.

### Example 6

The system (10) according to any of examples 1 through 4, wherein the EP map (302) is bipolar potential map.

### Example 7

The system (10) according to any of examples 1 through 6, wherein the ROIs (304) are tissue regions suspected of arrhythmogenic activity.

### Example 8

The system (10) according to any of examples 1 through 7, wherein the input device (110) comprises one of a touchscreen (27) and a computer mouse (112).

### Example 9

A method includes receiving or generating an electrophysiological (EP) map (302) comprising one or more regions of interest (ROIs) (304). A level of deemphasis (306, 308, 310) the EP map required outside (305) the ROIs (304) is received from a user. The EP map (302) outside (305) the ROIs (304) is deemphasized responsively to the required level (215, 306, 308, 310). The deemphasized EP map (302) is displayed to the user.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A system, comprising:
an input device (110); and
a processor (56), configured to:
receive or generate an electrophysiological (EP) map (302) comprising one or more regions of interest (ROIs) (304);
receive from a user, via the input device (112), a level of deemphasis (306, 308, 310) the EP map (302) required outside (305) the ROIs (304);
deemphasize the EP map outside the ROIs responsively to the required level (215, 306, 308, 310); and
display the deemphasized EP map to the user.

2. The system according to claim 1, wherein the processor (56) is configured to deemphasize the EP map (302) outside (305) the ROIs by darkening the EP map outside the ROIs.

3. The system according to any of claims 1 or 2, wherein the processor (56) is further configured to provide, using a display device (27) and the input device (110), a graphical user interface (GUI) (111) feature that lets the user adjust the level of deemphasis.

4. The system according to claim 3, wherein the GUI (111) feature comprises a sliding ruler (213) that lets the user adjust the level of the deemphasis.

5. The system according to any of claims 1-4, wherein the EP map (302) is a local activation time (LAT) map.

6. The system according to any of claims 1-5, wherein the EP map (302) is bipolar potential map.

7. The system according to any of claims 1-6, wherein the ROIs (304) are tissue regions suspected of arrhythmogenic activity.

8. The system according to any of claims 1-7, wherein the input device (110) comprises one of a touchscreen (27) and a computer mouse (112).

9. A method, comprising:
receiving an electrophysiological (EP) map (302) comprising one or more regions of interest (ROIs) (304);
receiving from a user a level of deemphasis (306, 308, 310) the EP map required outside (305) the ROIs;
deemphasizing the EP map outside the ROIs responsively to the required level (215, 306, 308, 310); and
displaying the deemphasized EP map to the user.

10. The method according to claim 9, wherein deemphasizing the EP map outside the ROIs comprises darkening the EP map outside the ROIs.

11. The method according to any of claims 9 or 10, and comprising providing a graphical user interface (GUI) (111) feature that lets the user adjust the level of deemphasis.

12. The method according to claim 11, wherein the GUI feature comprises a sliding ruler that lets the user adjust the level of the deemphasis.

13. The method according to any of claims 9-12, wherein the EP map (302) is a local activation time (LAT) map.

14. The method according to any of claims 9-13, wherein the EP map (302) is bipolar potential map.

15. The method according to any of claims 9-14, wherein the ROIs (304) are tissue regions suspected of arrhythmogenic activity.
